Europäisches Patentamt

European Patent Office

Office européen des brevets

(11) Publication number: **0 387 975**
**A1**

# EUROPEAN PATENT APPLICATION

(21) Application number: **90250065.1**

(51) Int. Cl.⁵: **C12N 5/00**

(22) Date of filing: **12.03.90**

(30) Priority: **16.03.89 JP 64910/89**
**06.04.89 JP 87588/89**
**23.05.89 JP 129968/89**
**25.09.89 JP 248967/89**

(43) Date of publication of application:
**19.09.90 Bulletin 90/38**

(84) Designated Contracting States:
**DE FR GB IT**

(71) Applicant: **W.R. Grace & Co.-Conn. (a Connecticut corp.)**
**Grace Plaza 1114 Avenue of the Americas**
**New York New York 10036(US)**

(72) Inventor: **Takezawa, Toshiaki**
**Nifty 34-302, 31-15 Higashi Naruse**
**Isehara-Shi, Kanagawa-Ken(JP)**
Inventor: **Mori, Yuichi**
**1-17-5-504 Higashi Ikuta, Tama-ku**
**Kawasaki-Shi, Kanagawa-Ken(JP)**
Inventor: **Sakai, Toshiya**
**201 Sato Mansion, 1-25-20 Goden**
**Hiratsuka-shi, Kanagawa-Ken(JP)**

(74) Representative: **UEXKÜLL & STOLBERG**
**Patentanwälte**
**Beselerstrasse 4**
**D-2000 Hamburg 52(DE)**

(54) Cell culture substrate cell sheet, cell cluster and preparations thereof.

(57) The invention provides cell culture substrates, cell sheets, cell clusters and preparations thereof using temperature-responsive polymeric compounds.

EP 0 387 975 A1

## CELL CULTURE SUBSTRATE, CELL SHEET, CELL CLUSTER AND PREPARATIONS THEREOF

Technical Field

This invention relates to a cell culture substrate. More particularly, this invention relates to a carrier suitable for cell culture where cell passage is required.

This invention further relates to a cell sheet and/or to a cell cluster and to the fabrication method of such a cell sheet and/or cell cluster. More particularly, this invention relates to a cell sheet and/or a cell cluster which have excellent self-supporting abilities and cellular functions and the method of fabrication of such a cell sheet and/or cell cluster. The cell sheet and/or cell cluster of this invention are useful for efficient production of cell products. They are also useful as a prosthesis which is used for repairing the damaged or diseased part of living tissue or as a simulating system to evaluate the effect of drugs and so on, on a living body.

Background Art

The major applications of the current mammalian cell culture technology are 1) bioreactors for production of cell products with physiological activities, 2) prosthesis for diseased or damaged living tissues or organs, 3) simulators to evaluate toxicity and activity of drugs and so on. The outline of the prior technology concerning the mammaliam cell culture is as follows.

The mammalian cells used in cell culture technology can be divided into two types. They are anchorage independent cells and anchorage dependent cells.

The former group, the anchorage independent cells, are cells which can perform their cellular functions, such as viability, proliferation and ability to produce substances, without a substrate that serves as a foothold for the cells. Typical examples are hybridomas formed from myeloma cells and lymphoma cells.

In contrast, the latter group, the anchorage dependent cells, are cells which normally cannot perform their cellular functions such as viability, proliferation and ability to produce substances, without a substrate that serves as a foothold for the cells. The majority of normal diploid cells, including primary cells, are anchorage dependent. Even many of the established cell lines are known to show anchorage dependency. For example, the established cell lines for production of useful cell products including cytokines, such as interferon and interleukin, differentiating factors such as erythropoietin, colony-stimulating factor, thrombopoietin, and tissue plasminogen activator and vaccines, are known to be anchorage dependent. In addition, most of the cells which are applicable to prosthesis for a diseased or damaged living body and to a simulator for evaluation of toxicity and activity of drugs, are conceived to be anchorage dependent. Therefore, establishment of culturing technology for the anchorage dependent cells is extremely important for these applications.

In general, to utilize cells for these applications it is important to culture the cells in a large quantity and at high density while keeping the cells at full functional level. However, animal cells, more so than microbial cells, are highly susceptible to the effects from deficiency of supply of nutrients such as oxygen, and to the effects of accumulation of metabolic waste products.

In the case of anchorage independent cells, suspension culture techniques are considered to be the best. If cells are cultured in suspension with agitation, waste materials can be removed quickly and nutrients can be supplied efficiently, and therefore it is easier to scale up the equipment for mass and high density culture.

However, in the case of anchorage dependent cells, it is not possible to use the suspension culture technique because the cells require a substrate for attachment. Therefore, different cell culture devices with substrate for cell attachment have so far been developed. For example, in experimental scale, dish-type, flask-type and plate-type devices have been most widely used. However, the above mentioned devices are not suitable for mass cell culture. Therefore, different ideas were conceived to increase the surface area of the substrate where the cells could adhere, relative to the total volume. For example 1) roller bottle type where bottles for cell culture are rotated to grow the cells on the entire surface of the wall, 2) multiple tray type where plates for cell adhesion are arranged parallel in the culture medium and the culture medium is circulated among the plates, 3) coil type where a plastic film formed into a coil is inserted into a cylindrical tube which is rotated in a lateral manner to adhere the cells, and then a culture medium is circulated among the film, 4) hollow fiber type where hollow fiber membranes possessing semipermeability are allowed to come in contact with the cells on the external surface of the hollow fibers and the culture medium is circulated through the interior of the hollow fiber to supply nutrients and remove waste materials through the hollow fiber membranes, 5) packed glass bead type where cells are in contact and adhered to the packed glass beads and the culture medium is circulated among them, 6) microbead type where microbeads are suspended in the culture medium to attach the cells on the surface of the microbeads which are agitated to culture the cells.

As mentioned above, prior attention has been mainly paid to morphological design of the cell culture device from the view point of effectiveness in nutrient supply and in waste removal. Recently however, it has been found that it is almost impossible to maintain cell viability and functions for a long period only by controlling the efficiency of nutrient supply and waste removal, but the cell culture substrate is a key to control the cell viability and functions for anchorage dependent cells. Therefore, research on the relationship between the property of cell culture substrate and cell functions has been actively carried out.

In the past, polystyrene is most widely used as a material of cell culture substrate because of its optical transparency, non-toxicity, excellent mechanical properties, good moldability and low price. However, the cell adhesion process which leads to the cell proliferation process is significantly inhibited on the surface of the polystyrene culture substrate because of its hydrophobicity. Therefore in order to improve the cell attachment and proliferation, the modified hydrophilic polystyrene which is endowed with negative charges by corona discharge treatment, has been developed and widely used as a cell culture substrate. However, it was found that the above-mentioned modification of polystyrene is still not enough for cells to express and maintain their specific functions.

Recently, the study to bring the cell culture substrate closer to the in vivo environment around the cell has started in order to improve cell functions such as attachment, proliferation, differentiation, and production ability of cell products. Namely, the study is to incorporate the substances which effectively control the cell functions into the cell culture substrate. The most typical substance to control the functions is extracellular matrix. Study of the function of extracellular matrix in vivo has progressed rapidly in recent years. It has become clear that it plays, not only a simple passive role such as supporting the cells and fixing the cells as known in the past, but also has a function in actively controlling or regulating cell functioning. Although a number of extracellular matrix components have been identified, the most important component is collagen. In addition it has been discovered that there are more than ten different types of collagen each of which is synthesized by a certain definite cell and is located in a certain tissue playing the role of controlling different cell functions. Even with the same type of collagen, modification by introducing a variety of functional groups or modification of higher order structure can cause different effects on the cell functions. As well as collagen, extracellular matrix components such as fibronectin, laminin, thrombospondin, vitronectin, proteoglycan and glycosaminoglycan have been identified. These have specific binding sites relative to the collagen and cell membrane and also play an important role in the cell attachment and proliferation.

Furthermore, except for the above-mentioned extracellular matrices, there are some other substances which effectively control cell functions such as attachment, proliferation and differentiation. They are gelatin which is a thermally degenerated collagen, lectins which bind specifically to sugar moiety on the cell membrane, anchorage oligopeptides which are the binding sites of anchorage proteins such as fibronectin, and adhesive protein isolated from a shellfish.

As examples of the culture substrates combined with these substances which control the cell functions, collagen-coated substrate (K. Yoshizato, et al., Annals of Plastic Surgery, 13, 9, 1984), fibronectin-coated substrate (F. Grinnell, Expl. Cell Res. 102, 51, 1976) and the substrate coated with adhesive protein of a shellfish (P. T. Picciano, et al., Developmental Biology 22, 24, 1986) have been developed, and some improvements in cell attachment and proliferation have been found.

Furthermore, recently the culture substrate coated with polystyrene containing galactose-derivative group as a side chain has been developed and some improvements in the attachment and life of hepatocytes have been recognized (T. Akaike, et al., Jpn. J. Artif. Organs, 17, 227, 1988). By using the cell culture substrates mentioned above, recently it has become possible to culture the cells which have not been able to attach and proliferate on the prior culture substrate such as glass or polystyrene.

However, despite these advancements in culture devices and substrates, the current cell culture technology has the following crucial problems still.

The distinct feature of the culture of anchorage dependent cells is that the cells stop further proliferation if the cells proliferate and completely cover the surface of the substrate. This is called contact inhibition. Therefore, the passage process, that is, the process to detach the cells from the old substrate and then to transfer the detached cells to a new substrate is necessary in order to continue the proliferation. In the past, proteolytic enzymes such as trypsin and collagenase, and EDTA as a calcium chelator were most commonly used for the cell detachment process. However, the prior cell detachment process, like

3

trypsinization, not only causes significant damage to the cell function, but also is a crucial obstacle to the cell culture process. The problems are as follows:

1) Prior detaching agents destroy not only the bonds between cells and the culture substrate but also bonds between neighboring cells. Three types of intercellular bonds, that is, tight junction, gap junction, and desmosome are known. The tight junction plays the role of barrier to the permeability of substances between the apical and basal sides. Through the gap junction, the exchange of substances and information is carried out between the neighboring cells, and by the desmosome, the cell assembly is mechanically supported. The cell is not able to be alive and functional alone, but the intercellular junctions enable the cell to express and maintain specific functions (B. Alberts, et al., "Molecular Biology of the Cell", 3rd edn., Garland Publishing Inc., New York & London, P. 673, 1983). Accordingly, the prior detaching agent causes crucial damage to the functions of the cultured cells by destroying completely the intercellular junctions formed in the culture process at the time of passage.

2) On the cell membrane there are many receptors for signaling molecules such as hormones, local chemical mediators, and neurotransmitters and the target cell communicates with the secreting cell through the specific reaction between the receptor and the signaling molecule. It has been found that the prior detaching agents destroy the receptors (e.g. C. Sung, et al., Biochem Pharmocol, 38, 696, 1989). Accordingly, the cells treated with the prior detaching agents cannot be controlled by the signaling molecules. This means that the cell loses its specific functions.

3) As a nutrient, the common culture medium contains serum which holds potent trypsin inhibitors. Therefore, prior to trypsinization the cells have to be washed thoroughly with a buffer solution in order to remove the trypsin inhibitors. This washing procedure not only complicates the operation, but also causes contamination which is a lethal problem in cell culture technology.

These major problems mean that even if the cells with the specific functions can be cultured by use of the sophisticate culture substrate combined with the extracellulrr matrix and also the effective design of a culture device for supply of nutrients and removal of waste, the recovery process of the cultured cell by use of the prior detaching agents markedly damages the cellular functions. Particularly, the deterioration of cell functions induced by the prior cell recovery process significantly reduces the ability of production of cell products. Also, by the cell recovery method, the self-supported cell assembly for a prosthesis cannot be acquired because the cell detaching agents such as trypsin completely break the cell assembly. Furthermore, the cells treated with the current recovery process are not applicable to simulators for evaluation of activity of the drugs, since the membrane-bound receptors responsive to the drugs are completely digested by trypsin. In addition, the prior recovery process brings a crucial shortcoming particularly to the mass cell culture technology. Excessively low initial cell concentrations of anchorage dependent cells in culture medium is said to retard the proliferation of cells and the ability to produce substances even if the cells adhere to the substrate. Particularly for the cases with primary cells or normal diploid cells which are difficult to harvest, cultivation in a large volume of culture media from the start will reduce the cell concentration excessively, and therefore the concentration of the cells has to be increased by repeating the cell culture in steps using a culturing device having a smaller capacity. This fact means that in the mass cell culture process, a lot of repeated cell recovery processes are necessary. Accordingly, the effect of the prior cell detachment procedure is such more crucial compared to a small quantity cell culture process.

The objective of this invention is to provide a cell culture substrate which can solve the problems such as deterioration of cellular functioning, complication of operation, and risk of contamination, which accompany the cultured cell recovery process by use of prior cell detaching agents such as trypsin, collagenase, EDTA and so on.

Another objective of this invention is to provide a cell sheet and/or a cell cluster which are recovered without cell function damage caused by prior detachment agents. These cell sheets and/or cell clusters are available for production of cell products, prosthesis for diseased or damaged living tissues or organs and a simulator to evaluate toxicity and activity of substances such as drugs.

Description of the Invention

The cell culture substrate of this invention comprises a temperature-responsive polymeric compound that has a lower LCST than the cell culture temperature. Here, LCST or lower critical solution temperature, is a transition temperature for hydration and dehydration of the polymeric compound. The cell culture substrate of this invention can additionally comprise substances which effectively control cell functions such as attachment, proliferation and differentiation.

The cell culture substrate of this invention can solve the problems such as deterioration of cell functions, risk of contamination and laboriousness of operation that accompany cell recovery and passage which are the problems of the cell culture technique of the prior art.

This invention provides: 1) a substrate made from a temperature-responsive polymeric compound having lower LCST than the culture temperature; 2) a carrier formed by coating the said polymeric compound on the supporting material; 3) a carrier made by graft-polymerizing the said polymeric compound on the surface of the supporting material; 4) a carrier which consists of microbeads made from the said polymeric compound having a crosslinked structure; 5) a substrate made from a mixture of the said polymeric compound and substances which effectively control cell functions such as attachment, proliferation and differentiation; 6) a carrier formed by coating the mixture of the said polymeric compound and substances which effectively control cell functions on the supporting material; 7) a carrier formed by laminating a support with the said polymeric compound layer and a layer of substances which effectively control cell functions in sequence; 8) cell sheets and/or cell clusters formed by any of the foregoing.

The temperature-responsive polymeric compound having lower LCST than the culture temperature to be used as substrate in this invention is in a solid state which the cells can utilize as an anchor to adhere and proliferate at cell culture temperature, and will become a soluble state by reducing the temperature below the LCST to permit detachment of cells from the substrate for passage. In addition, in a carrier grafted with the temperature-responsive polymeric compound, the exchange between hydrophilic and hydrophobic states induced by temperature change will detach the cells. In a microbead carrier made from the temperature-responsive polymeric compound with crosslinked structure, the exchange between hydrophilic and hydrophobic states, and between swelling and deswelling states will detach the cells.

Examples of temperature-responsive polymeric compounds that can be used as a substrate in this invention are poly-N-substituted (meth)acrylamide derivatives and their copolymers, polymethylvinyl ether, polyethylenoxide, etherized methylcellulose, and partially acetylated polyvinyl alcohol. Particularly preferred compounds are poly-N-substituted acrylamide derivatives or poly-N-substituted methacrylamide derivatives or their copolymers.

For example, poly-N-isopropylacrylamide (PNIPAAm) is a polymeric compound which shows a negative temperature coefficient of solubility in water (Heskins, M., et al., J. Macromol. Sci.-Chem., A2(8), 1441, 1968). The hydrate (oxonium hydroxide) which depends on the hydrogen bonding formed at a lower temperature between a water molecule and the polymer molecule will decompose at a higher temperature, so that polymers aggregate by dehydration to form a precipitate. Thus, the transition temperature of this hydration and dehydration is called "lower critical solution temperature" or LCST. Thus above the LCST, the said polymer aggregates to form a solid state. But at a temperature lower than the LCST, the polymer dissolves in water.

The present invention takes advantage of such properties of the temperature-responsive polymeric compounds and completes a substrate for cell culture that can attach or detach the cultured cells by merely changing its temperature.

Appropriate temperature-responsive polymeric compounds to be used as substrates of this invention are indicated below, but this invention is not limited to these examples. The LCST of these polymers rise with the sequence of polymers listed below.

Poly-N-acryloyl piperidine, poly-N-n-propyl methacrylamide, poly-N-isopropyl acrylamide, poly-N,N-diethyl acrylamide, poly-N-isopropyl methacrylamide, poly-N-cyclopropyl acrylamide, poly N-acryloyl pyrrolidine, poly-N,N-ethylmethyl acrylamide, poly-N-cyclopropyl methacrylamide, poly-N-ethyl acrylamide.

The aforesaid polymers may be homopolymers or copolymers with other monomers. Any hydrophilic monomers or hydrophobic monomers can be used as the monomer for copolymerization. Generally speaking, copolymerization with hydrophilic monomer will raise the LCST, and copolymerization with hydrophobic monomer will depress the LCST. With a proper selection of monomers, a copolymer with a desired LCST can be achieved.

Examples of hydrophilic monomers are N-vinylpyrrolidone, vinylpyridine, acrylamide, methacrylamide, N-methyl acrylamide, hydroxyethyl methacrylate, hydroxyethyl acrylate, hydroxymethyl methacrylate, hydroxymethyl acrylate, acrylic acid and methacrylic acid having acidic groups and its salts, vinyl sulfonic acid, styrylsulfonic acid and N,N-dimethylaminoethyl methacrylate, N,N-diethylaminoethyl methacrylate, and N,N-dimethylaminopropyl acrylamide having basic groups and their salts, but it is not limited to these compounds.

Examples of hydrophobic monomers are acrylate derivatives and methacrylate derivatives such as ethyl acrylate, methyl methacrylate and glycidyl methacrylate and so on, N-substituted alkyl (meth) acrylamide derivatives such as N-n-butyl (meth) acrylamide and so on, vinyl chloride, acrylonitrile, styrene, and vinyl acetate and so on, but it is not limited to these compounds.

In general, a copolymer with a monomer having a basic group is desirable because it may enhance

5

adhesiveness of cells to the substrate by electrostatic interaction with the negatively charged cell membrane.

On the other hand, in this specification, the substances which effectively control cell functions such as attachment, proliferation and differentiation refer to the extracellular matrix, gelatin, lectins, anchorage oligopeptides which are the binding sites of anchorage proteins such as fibronectins, and adhesive protein isolated from a shellfish and so on. The extracellular matrix refers to the substances existing among cells within the living body. Specifically collagen, fibronectin, laminine, vitronectin, proteoglycan, glycosaminoglycan and thrombospondin and so on are included.

In addition to such properties of temperature-responsive polymeric compounds, the present invention focuses on the substances which effectively control cell functions and has perfected a cell culture substrate that can anchor and proliferate specific cells with a high degree of cell functioning by combining the said polymeric compound and the cell controlling substances.

There is no particular limitation with the shape of the substrate of this invention, and it can be of various shapes such as film, sheet, granule, fiber, flake, sponge, microbeads and so on. As a substrate for the cell culture, film, sheet, granules or fibers are particularly desirable. In the case of microbeads, the preferred particle size is 50-300 microns.

Ordinary molding methods of polymeric compound can be used to form these shapes. For example, a polymer with or without substances such as extracellular matrix is dissolved in water or organic solvent and the solution is formed into a film or sheet by a standard solvent casting technique. The liquid polymer mixture, while being cooled below the LCST, is extruded through an orifice into water or water-immiscible organic solvent at a temperature higher than the LCST, to form a film, grain, particle or fiber. The polymer may be formed into any desired shapes by suspension polymerization, precipitation polymerization, or molding directly into grains. To fabricate microbeads the polymer is dropped in water-immiscible organic solvent to form microbeads which are then insoluble.

To fabricate a carrier of this invention, a supporting material having a desired shape is prepared ahead of time, and then the polymer, with or without substances such as extracellular matrices, is coated on the surface of that supporting material by a standard method. The method of coating a thin layer of an aqueous or organic solution of the said polymeric compound, with or without substances such as extracellular matrices, on the surface of the supporting material by solvent casting methods and drying that film is particularly simple and effective. To produce a carrier that has a layer of polymeric compound and a layer of substances such as extracellular matrix on the support, the carrier can be prepared by a method by which each layer is coated in a sequential manner.

For graft-polymerizing the polymeric compound on the surface of a supporting material, one can select a polymeric compound having a desired LCST and a copolymerization grafting technique with various types of monomers can be used to regulate it to any desired LCST. When performing copolymerization graft technique, hydrophilic monomers or hydrophobic monomers can be used. In general, copolymerization with hydrophilic monomers causes the LCST of the graft copolymer to rise. Conversely, copolymerization with hydrophobic monomers will depress the LCST. Types of hydrophilic monomers and hydrophobic monomers that can be used in this invention are listed above.

Materials which have been conventionally used as substrate for cell culture are desirable as the supporting material in the case of graft-polymerizing the polymeric compound onto the surface. Examples are glass, polystyrene, polycarbonate, polymethyl methacrylate, polystyrene, polypropylene, polyethylene, polyesters, polyamides,, polyvinylidene fluorides, polyoxymethylene, polyvinyl chloride, polyacrylonitrile, polytetrafluorethylene, polydimethylsiloxane, cellulosic polymers, crosslinked dextran, crosslinked polyacrylamides, collagen and so on, but they are not limited to these compounds.

An appropriate method of grafting the temperature-responsive monomer to the supporting material can be selected based on the types of materials used and their shape.

Low temperature plasma polymerization technique can be used appropriately when the supporting material is a sheet, film or flat membrane. This method can graft-polymerize only on the surface of the supporting material without damage to the bulk property and is applied to polymers which have difficult forming radicals by usual methods such as polypropylene, polyethylene, polytetrafluoroethylene, polydimethylsiloxane, polyesters, polycarbonates, polymethyl methacrylate and so on. If the supporting material is a hollow fiber membrane or microbeads, the ozone oxidation method and cerium ion method are most appropriate. Particularly, these methods are most suitable as a graft-polymerization method for the hollow fiber membrane made of cellulosic polymer, or for microbeads made of crosslinked dextran, crosslinked polyacrylamide, or collagen.

A carrier prepared by graft-polymerizing the said polymeric compound on the surface of a support can have various shapes based on the cell culture method and property of the support and so on. Examples are 1) sheets or films, such as culture dishes, 2) hollow fiber membranes or flat membrane types, and 3) microbeads, and so on.

Besides, graft polymerization methods using ultraviolet light, x-ray, gamma-ray or electron beam can be used.

Crosslinking structures can be formed by using a method of introducing the crosslinking structure during polymerization of the monomer or by using a method of introducing the crosslinking structure after completion of polymerization. Either method can be used in this invention.

Specifically, the former method is carried out by copolymerizing bifunctional monomers. For example, N,N-methylenebisacrylamide, hydroxyethyl dimethacrylate or divinylbenzene can be used. With the latter method, it is common to crosslink the molecules by light, electron beam and gamma-irradiation.

On the other hand, the present invention also takes advantage of the properties of temperature-responsive polymeric compounds and has completed a cell sheet and/or cell cluster. After forming a cell monolayer on the substrate of the temperature-responsive polymeric compound with or without substances such as the extracellular matrix by culturing cells at a higher temperature than the LCST, the cell sheet can be prepared by detaching it from the substrate by lowering the temperature below the LCST. In order to recover a cell sheet formed on the prior substrate, cell detaching agents such as trypsin are necessary. The prior cell detaching agents destroy not only junctions between cells and the substrate but also junctions between individual cells. Accordingly, it was impossible to prepare an excellent self-supporting cell sheet by the prior art. Furthermore, the prior cell detaching agents significantly damaged the cell membrane and membrane-bound receptors.

On the other hand, in this invention it is possible to detach and recover a cell sheet from the substrate by merely changing the temperature instead of using the prior detaching agents. This invention first enables the formation of an excellent self-supported cell sheet which was almost impossible to make by the proir detaching agents. Also, this invention can keep viability and cell functions of the cell sheet due to no use of the prior detaching agents. This invention also enables the formation of an excellent self-supported cell sheet which was almost impossible to make by the prior detaching agents. Furthermore, this invention can significantly simplify the prior complex cell detaching process where the cell washing process and trypsin adding process are necessary. This means that this invention can markedly reduce the possibility of contamination which is a lethal problem in cell culture technology.

The cell detached from the substrate by the method in this invention were sheet-like immediately after the detachment, but if the cell sheet was transferred to a non-anchorage hydrophobic dish, the cell sheet gradually rolled up and finally changed to a cell cluster. It is a matter of course to be able to keep sheet-like without changing to a cluster if the circumference of the cell sheet is fixed during the detachment process.

The cell density of the thus formed cluster can be in the order of $10^9$ cells/ml. This cell density is about 100 times higher than the maximum cell density that can be attained by prior culture (order of $10^7$ cells/ml). Thus, although it is a simple calculation, it means that the scale of the equipment for production of cell products can be reduced to about a hundredth.

In the prior art, it was impossible to control the size of the cell cluster and also to produce the cluster in mass, since the prior cell cluster was formed by accidental detachment from the substrate (Koide, N. et al., Jpn. J. Artif. Organs 17 (1), 179, 1988). In this invention, however, it is easy to control the size of the cluster by changing the surface area of the culture substrate where the cell proliferated and covered before detachment and also to make the cluster in mass. In this invention, the size of the cluster ranges from several microns to several mm. Particularly, this invention is more suitable for production of the larger clusters which were not able to be prepared by the prior art. The larger clusters are effective as the prosthesis for diseased or damaged living tissue or organs.

In addition, when the cell cluster was transferred to a new hydrophilic dish, after the cell cluster was preserved on a non-anchorage hydrophobic dish in a $CO_2$ incubator for a long period using a common culture medium, the cell cluster started to reattach and reproliferate on the hydrophilic dish. This evidence shows that the cell cluster can keep its viability and cell function for a long period. This is because the intercellular junctions and membrane-bound receptors of the cell cluster are kept intact by the detachment method in this invention.

The cell culture substrate of this invention enables a very simple cell recovery by replacing the prior cell detaching agents with mere change in temperature and reduce the complexity and risk of contamination of the prior cell culture technology. In addition, the cell sheet and/or cell cluster of this invention showed high cell density, high cellular functioning and excellent self-supporting properties. This means that the cell sheet and/or cell cluster of this invention are strongly available for 1) bioreactors for production of cell products, 2) prosthesis for diseased or damaged living tissues or organs, and 3) simulators to evaluate toxicity and activity of drugs and so on.

The following abbreviations have been used throughout in describing the invention.

AIBN - 2',2'-azobisisobutylnitrile

$cm^2$ - square centimeter

$CO_2$ - carbon dioxide

°C - degrees centigrade

DMEM - Dulbecco's modified Eagle's medium

FCS - fetal calf serum

g - gram

LCST - lower critical solution temperature

ug - microgram

ul - microliter

ml - milliliter

mm - millimeter

mM - millimolar

n-BMA - n-butyl methacrylate

PBS - phosphate buffered solution

% - percent

PNIPAAm - poly-N-isopropyl acrylamide

THF - tetrahydrofuran

Examples and comparative examples are illustrated below to explain further and embody this invention.

## Example 1

N-Isopropyl acrylamide monomer (NIPAAm, Eastoman Kodak Co.) 50g was dissolved in 500ml benzene. Using 0.2g AIBN as the polymerization initiator, polymerization was carried out at 60°C for 12 hours in a stream of nitrogen gas with agitation. The obtained polymer precipitated in benzene and after decantation the precipitation was dissolved in THF and purified using ethyl ether. LCSTs of the thus obtained PNIPAAm in different solutions (polymer concentration: 1%) which were measured by turbidimetric method are shown in Table I. As shown in Table I, it was found that PNIPAAm shows the sharp conversion between hydrophobic and hydrophilic states not only in water but also in calf serum and culture medium.

### Table I

| LCST of PNIPAAm in different solutions | |
| --- | --- |
| Solution | LCST (°C) |
| Distilled Water | 31.8 ± 0.1 |
| PBS | 28.9 ± 0.1 |
| Calf Serum | 28.2 ± 0.1 |
| Culture Medium | 28.6 ± 0.1 |

## Example 2

NIPAAm 50g, n-butyl methacrylate (n-BMA) 3.3g and AIBN 0.21g as the polymerization initiator, were dissolved in 500ml of THF. Polymerization was carried out at 50°C for 12 hours in a stream of nitrogen gas with agitation. After concentrating the reaction mixture twofold in an evaporator, it was precipitated and purified with ethyl ether and dried under vacuum to obtain a flaky polymer (Cop.(NIPAAm/BMA)-1). In addition, by using NIPAAm 50g, n-BMA 6.6g and AIBN 0.21g, Cop.(NIPAAm/BMA)-2 was obtained by the same method as the Cop.(NIPAAm/BMA)-1. LCSTs of the obtained polymers in PBS and calf serum were measured by turbidimetric method and are listed in Table II. As shown in Table II, with the comonomer ratio of n-BMA, the LCST of the copolymer significantly decreased.

Table II

| LCST of Cop(NIPAAm/BMA) in PBS and calf serum | | |
| --- | --- | --- |
| | LCST ($^{\circ}$C) | |
| Polymer | PBS | Calf Serum |
| PNIPAAm | 28.9 ± 0.1 | 28.2 ± 0.1 |
| Cop.(NIPAAm/BMA)-1 | 19.0 ± 0.1 | 20.5 ± 0.4 |
| Cop.(NIPAAm/BMA)-2 | 7.8 ± 0.3 | 13.8 ± 0.2 |

Example 3

By dissolving PNIPAAm and NIPAAm monomer which were synthesized and used in Example 1, in DMEM containing 10% FCS, 1.0% PNIPAAm and 1.0% NIPAAm, solutions were prepared for cytotoxicity tests. For control, DMEM containing 10% FCS was used. Then, human dermal fibroblasts were dispersed in PNIPAAm, NIPAAM and control solutions so as to form a cell density of about $1 \times 10^5$/ml and each solution of 2 ml was poured into the plastic 35 mm cell culture dishes (Falcon Co.). The cells were cultured at 25$^{\circ}$C in a $CO_2$ incubator (air/5% $CO_2$). After culturing for one or three days, attachment and proliferation of the cells were examined by a phase contrast microscope. The degree of attachment and proliferation were used as a measure of cytotoxicity. The results are shown in Table III. As shown in Table III, no cytotoxicity of PNIPAAm was observed, although strong cytotoxicity was recognized in the NIPAAm monomer.

Table III

| Cell attachment and proliferation | | |
| --- | --- | --- |
| | Cell attachment and Proliferation | |
| Substance | 1-day culture | 3-day culture |
| Control PNIPAAm NIPAAm | ◯ ◯ × | ◎ ◎ × |
| ◎ : Excellent ◯ : Good × : Poor | | |

Example 4

The cytotoxicity of Cop.(NIPAAm/BMA)-1 which was synthesized in Example 2 was evaluated by the same method as Example 3 and shown in Table IV. Here, the cell culture was carried out at 17$^{\circ}$C so that the polymer can dissolve in the culture medium. As shown in Table IV, no cytoxicity was observed in Cop.-

(NIPAAm/BMA)-1.

Table IV

| Cell attachment and proliferation | | |
|---|---|---|
| | Cell Attachment and Proliferation | |
| Substance | 1-day culture | 3-day culture |
| Control<br>Cop.(NIPAAm/BMA)-1<br>Mixture of NIPAAm and n-BMA monomers | △<br>△<br>× | ○<br>○<br>× |
| ○ : Good<br>△ : Not Very Good<br>× : Poor | | |

## Example 5

An aqueous solution (0.5%) of PNIPAAm which was synthesized in Example 1 was prepared and was sterilized by autoclaving (121°C, 20 minutes) and then cooled to redissolve the polymer. To coat, after pouring the 0.5% aqueous PNIPAAm solution into the plastic 35 mm cell culture dish (Falcon Co.) and coating uniformly, excess solution was discarded and the dish was dried in a clean hood at room temperature. The above-mentioned process was performed aseptically. Human dermal fibroblasts which were used in Example 3 were dispersed in DMEM containing 10% FCS to form a cell density of about 2 x $10^5$/ml. Two mililiters of the cell suspension kept at 37°C was poured into the PNIPAAm-coated dish which was kept at 37°C. The cells were cultured at 37°C in a $CO_2$ incubator (air/5% $CO_2$). After culturing for 7 days, colony formation was sporadically found on the bottom of the dish and the outside of the dish was cooled to 15°C. By a phase contrast microscope it was observable that the colony which attached on the dish spontaneously detached from the bottom.

## Example 6

An aqueous solution (0.5%) of Cop.(NIPAAm/BMA)-1 which was synthesized in Example 2 was prepared and was sterilized by autoclaving (121°C, 20 minutes) and then cooled to redissolve the polymer. Using the solution, Cop.(NIPAAm/BMA)-1-coated dish was prepared by the same method as Example 5. Then the human dermal fibroblasts were cultured on the coated dish and the colony formation similar to Example 5 was found after 7 days. When the dish was cooled to about 10°C, the colony was found to spontaneously detach from the dish surface.

## Example 7

An aqueous solution (0.5%) of PNIPAAm which was synthesized in Example 1 was prepared and sterilized by filtration through a 0.45 micron filter. This solution was mixed with an equal volume of 0.5% Type I collagen solution which was solubilized from cow skin by pepsinization (Sterilized, Koken K.K.) to prepare a solution containing 0.25% PNIPAAm and 0.25% collagen as the final concentrations. LCST of the mixture solution which was measured by turbidimetric method was about 32°C. This solution was poured into the plastic 35mm cell culture dish (Falcon Co.) and dried in a clean hood at room temperature. The above-mentioned process was performed asceptically. Thus, dishes which were coated with a mixture of collagen and PNIPAAm in equal volume in different thicknesses were prepared. Here, the coating thickness was controlled by the volume of the mixture solution poured into the dish. Then, the human dermal fibroblasts which were used in Example 5 were dispersed in DMEM containing 10% FCS to form a cell density of about $2 \times 10^5$/ml. Two mililiters of the cell suspension kept at 37°C was poured into the dishes coated with the mixture of PNIPAAm and collagen in different thicknesses which were kept at 37°C. The cells were cultured at 37°C in a $CO_2$ incubator (air/5% $CO_2$) for 5 days. The relationship between cell proliferation and the thickness of the coating layer is shown in Table V. On the other hand, the relationship between the thickness of the coating layer and the cell detachment which was observed by a phase contrast microscope when the dishes were transferred from 37°C to room temperature, is also shown in Table V. As shown in Table V, the cell proliferation was excellent independent of the thickness and the cell detachment was improved with the thickness reaching a maximum at a thickness of more than 0.7 um.

For a comparative example, using a non-coated 35 mm plastic dish (Falcon Co.), the human dermal fibroblasts were cultured in the above-mentioned manner. After the cells fully covered the dish, the dish temperature was cooled from 37°C to room temperature, but no detachment of cells was observed. Therefore, the following prior cell detachment process was carried out. The culture medium was discarded from the dish and 2ml of PBS was poured into the dish to wash the surface of the cells for removal of trypsin inhibitor contained in the medium and then the PBS was discarded. Then 2 ml of trypsin/EDTA solution (0.05% trypsin, 0.5 mM EDTA) was added to wash the surface of the cells, the trypsin/EDTA solution was discarded. Again 2 ml of fresh trypsin/EDTA solution was added and the solution except 0.5 ml was discarded. Then the dish was incubated at 37°C for 10 minutes. Using a microscope, complete detachment of cells from the dish was confirmed but the detached cells were isolated from each other and did not make a cell sheet. Also, the prior detachment process using detaching agents consists of a lot of procedures and was significantly complex compared to the current process of merely changing the temperature.

Table V.

| Dependency of cell proliferation and cell detachment upon the thickness of coating layer of mixture of PNIPAAm and collagen (1/1) | | |
|---|---|---|
| Thickness (um) | Cell Proliferation | Cell Detachment |
| 0 | ◎ | × |
| 0.2 | ◎ | ○ |
| 0.5 | ◎ | ○ |
| 0.7 | ◎ | ◎ |
| 0.9 | ◎ | ◎ |
| ◎ : Excellent  ○ : Good  × : Poor | | |

Example 8

The aqueous solutions of the different mixtures of PNIPAAm and collagen which were used in Example 7 were prepared. The composition ratio of the collagen to PNIPAAm are shown in Table VI. The dishes coated with these aqueous solutions in a thickness of about 0.9 um were prepared by the method similar to Example 7. The human dermal fibroblasts were cultured in a similar manner to Example 7. The relationship of the composition ratio of collagen to PNIPAAm with cell proliferation and cell detachment was measured and is shown in Table VI. The cell proliferation rose with the content of collagen and cell detachment

11

improved with the content of PNIPAAm. The compositiom ratio of collagen to PNIPAAm which is suitable to both cell proliferation and detachment, ranges from about 0.1/1.0 to 2.0/1.0.

Table VI

| Dependency of cell proliferation and cell detachment upon composition ratio of collagen to PNIPAAm. | | |
|---|---|---|
| Composition Ratio of Collagen to PNIPAAm | Cell Proliferation | Cell Detachment |
| 0.0/1.0 | △ | ◎ |
| 0.1/1.0 | ○ | ◎ |
| 0.5/1.0 | ○ | ◎ |
| 1.0/1.0 | ◎ | ◎ |
| 2.0/1.0 | ◎ | ◎ |
| 10.0/1.0 | ◎ | △ |
| 1.0/0.0 | ◎ | × |
| ◎ : Excellent ○ : Good △ : Not Good × : Poor | | |

## Example 9

Using the dish coated with the mixture of collagen and PNIPAAm (1.0/1.0) at a thickness of about 0.9 um in Example 8, the endothelial cells isolated from a calf pulmonary artery (CPAE, American Type Culture Collection) were cultured. The CPAE cells were dispersed in DMEM containing 10% FCS to form a cell density of about $2 \times 10^5$/ml. Two mililiters of the cell suspension kept at 37°C was poured into the aforesaid coated dish kept at 37°C and the cells were cultured at 37°C in a $CO_2$ incubator (air/5% $CO_2$) for 4 days. The cells proliferated and fully covered the dish. When the dish was withdrawn from the 37°C incubator and left at an ambient temperature, it was found by phase contrast microscope examination that the CPAE cells completely detached from the dish surface so as to form a cell sheet.

## Example 10

Using the dish coated with the mixture of collagen and PNIPAAm (2.0/1.0) in a thickness of about 0.9 um in Example 8, human epidermal cells (including keratinocytes) were cultured. A thin piece of human skin scraped by a dermatome was treated with trypsin to isolate epidermal cells, which were dispersed in F-12 media supplemented with hydrocortisone and adenine, so as to form a concentration of about $3 \times 10^5$/ml. Two mililiters of the cell suspension kept at 37°C was poured into the aforesaid coated dish kept at 37°C and the cells were cultured at 37°C in a $CO_2$ incubator (air/5% $CO_2$) for 7 days. The medium was replaced every 2 days, using culture medium kept at 37°C. After a 7-day culture, the cells proliferated and fully covered the dish. The outside of the dish was soaked in 10°C water to detach the cells from the bottom of the dish and thus a cell sheet was fabricated. The cell sheet was recovered by merely changing the temperature and the operation was very simple.

## Example 11

An aqueous solution (0.5%) of atactic polymethylvinyl ether (Tokyo Kasei K.K.) was prepared. The solution was sterilized by autoclaving (121°C, 20 minutes) and then cooled to redissolved the polymer. The LCST of the atactic polymethylvinyl ether was about 35°C in PBS as measured by turbidimetry. This solution was mixed with equal vomume of 0.5% collagen solution used in Example 7 to prepare a solution containing 0.25% polymethylvinyl ether and 0.25% collagen as the final concentration. By a method similar to Example 7, the dish coated with the mixture to a thickness of about 1 um was prepared. Then the human dermal fibroblasts used in Example 3 were dispersed in DMEM to form a cell density of about $2 \times 10^5$/ml. Two mililiters of the cell suspension kept at 40°C was poured into the aforesaid dish kept at 40°C and the cells were cultured at 40°C in a $CO_2$ incubator (air/5% $CO_2$) for 5 days. The cells covered the dish and the dish was withdrawn from the 40°C incubator and left at an ambient temperature. The cells were spontaneously detached from the dish and the cell sheet was suspended in the medium.

## Example 12

An aqueous solution (0.5%) of PNIPAAm which was synthesized in Example 1 was prepared, and sterilized by filtration through a 0.45 micron filter. Then this solution was mixed with an equal volume of 0.05% gelatin aqueous solution (Iwaki Glass K.K.) and 400 ul of the aforesaid mixture solution was poured into a plastic 35 mm cell culture dish (Falcon Co.) and dried in a clean hood at an ambient temperature. The above-mentioned procedure was carried out aseptically. Using the dish coated with the mixture of gelatin and PNIPAAm, the human dermal fibroblasts were cultured by the same method as Example 7. After the cell proliferation, the outside of the dish was cooled to about 10°C and the cell sheet was able to be detached from the dish and recovered.

## Example 13

An aqueous 5% solution of PNIPAAm used in Example 1 was prepared and sterilized by filtration through 0.45 micron filter. Then this solution was mixed with aqueous solutions of fibronectin isolated from calf plasma (Nitta Gelatin K.K.) so as to form mixture solutions with different compositions. The plastic 35 mm dishes (Falcon Co.) were coated with these mixture solutions with different compositions and air-dried at room temperature in a clean hood. These procedures were carried out aseptically. By the same method as Example 8, the human dermal fibroblasts were cultured on the dishes with different compositions and the relationship of the composition of fibronectin and PNIPAAm with cell proliferation and cell detachment was studied (Table VII). As shown in Table VII, cell proliferation improved with the composition ratio of fibronectin to PNIPAAm, but the detachment was poorer than collagen.

Table VII

| Dependency of cell proliferation and detachment upon composition ratio of fibronectin to PNIPAAm (thickness: 0.9 um) | | |
|---|---|---|
| Composition Ratio of Fibrinogen to PNIPAAm | Cell Proliferation | Cell Detachment |
| 0.01/1.0 | Δ | Δ |
| 0.04/1.0 | ○ | Δ |
| 0.08/1.0 | ◎ | × |
| ◎ : Excellent<br>○ : Good<br>Δ : Not Good<br>× : Poor | | |

## Example 14

The aqueous solution of PNIPAAm used in Example 13 was mixed with an aqueous solution of adhesive protein isolated from a shellfish, Cell-Tak® (Collaborative Research Inc.) in different compositions. The plastic 35 mm dishes were coated with these mixture solutions to a thickness of about 0.9 μm and by the same method as Example 13 the relationship between the composition ratio of Cell-Tak® to PNIPAAm and cell proliferation and detachment was measured (Table VIII). As shown in Table VIII, the result was almost the same as that of fibronectin although the cell proliferation was slightly inferior to that of fibronectin.

Table VIII

| Dependency of cell proliferation and detachment upon composition ratio of Cell-Tak® to PNIPAAm (thickness: 0.9 μm) | | |
|---|---|---|
| Composition Ratio Cell-Tak® to PNIPAAm | Cell Proliferation | Cell Detachment |
| 0.01/1.0 | Δ | Δ |
| 0.04/1.0 | Δ | Δ |
| 0.08/1.0 | O | × |
| ◎ : Excellent O : Good Δ : Not Good × : Poor | | |

## Example 15

The aqueous solution of PNIPAAm used in Example 13 was mixed with an aqueous solution of one species of lectin, concanavalin A (Hohnen Co.) in different compositions. The plastic 35 mm dishes were coated with these mixture solutions to a thickness of about 0.9 um and by the same method as Example 13, the relationship between the composition ratio of concanavalin A to PNIPAAm and cell proliferation and cell detachment was measured (Table IX). As shown in Table IX, although the cell attachment was observed, no cell proliferation was really recognized.

Table IX

| Dependency of cell proliferation and detachment upon composition ratio of concanavalin A to PNIPAAm (thickness: 0.9 um) | | |
|---|---|---|
| Composition Ratio of Concanavalin A to PNIPAAm | Cell Proliferation | Cell Detachment |
| 0.01/1.0 | Δ * | Δ |
| 0.04/1.0 | Δ * | Δ |
| 0.08/1.0 | Δ * | Δ |
| 0.5 /1.0 | ○ * | Δ |

*: Although cell attachment was good, no cell proliferation was really observed.
◎ : Excellent
○ : Good
Δ : Not Good
× : Poor

## Example 16

First, using 0.5% aqueous solution of PNIPAAm prepared in Example 1, the plastic 35 mm dish was coated with PNIPAAm to a thickness of about 0.9 um and then 400 ul of aqueous solution of fibronectin (concentration 0.1 mg/ml) kept at 37° C and used in Example 13 was poured into the aforesaid PNIPAAm-coated dish kept at 37° C and the dish was air-dried asceptically in a 37° C incubator. By this method, the dish having a laminated coating composed of a PNIPAAm layer and subsequently of a fibronectin layer whose fibronectin density is about 5 ug/cm², was prepared. Using this coated dish, by the same method as Example 7, the cell proliferation and cell detachment profiles were evaluated. As a result, the cell proliferation and simultaneously the cell detachment were excellent.

## Example 17

A commercial available polyethylene terephtalate (PET) film (size: 5 cm x 5 cm, thickness: 100 um, Toray Ind. Inc.) was inserted into a chamber of plasma irradiation apparatus with an internal electrode (Samco International K.K.). After the interior of the chamber was evacuated to 0.8 Torr, argon gas was introduced into the chamber at a flow rate of 30ml/min and the film was irradiated with plasma (output = 100 Watts, frequency = 13.56 MHz) for 15 seconds. Immediately thereafter, 5% aqueous solution of NIPAAm monomer was introduced to the chamber so as to soak the film in the solution and the polymerization was carried out at room temperature for 16 hours. In advance, from the monomer solution, contaminated air was completely removed by bubbling with argon. After washing the treated film thoroughly with water, it was dried under vacuum at an ambient temperature.

For controls, without introducing NIPAAm monomer into the chamber, the PET film was treated by plasma irradiation in similar conditions. Also, the PET film merely coated with PNIPAAm was prepared by a solvent casting method using PNIPAAm aqueous solution. The contact angles of the above-mentioned films against water 10° C and 40° C were measured and listed in Table X. As shown in Table X, in untreated film and plasma-treated film, no change in contact angle was observed between at 10° C and at 40° C, but in PNIPAAm coated film and PNIPAAm plasma-grafted film, significant differences in contact angle were found. Both films showed hydrophilicity at 10° C and hydrophobicity at 40° C.

Table X

| Contact angles of films | | |
|---|---|---|
| | Contact Angle (°) | |
| Film Species | 10°C | 40°C |
| Untreated Film | 77.0 ± 1.6 | 77.0 ± 2.3 |
| Plasma-Treated Film | 50.0 ± 1.7 | 50.1 ± 0.5 |
| PNIPAAm Coated Film | 43.7 ± 3.4 | 64.1 ± 2.9 |
| PNIPAAm Plasma-Grafted Film | 48.4 ± 4.9 | 60.7 ± 6.8 |

## Example 18

50 g of NIPAAm, 3.3 g of n-BMA and 1.5 g of N,N'-methylenebisacrylamide were dissolved in 100 ml of water. This solution was suspended in 500 ml of hexane. Using ammonium persulfate 0.13 g and tetramethylethylenediamine 0.13 g as the polymerization initiator, they were polymerized at an ambient temperature for 24 hours in a stream of nitrogen gas with a constant agitation at 300 rpm, to acquire crosslinked microbeads. The microbeads were washed with 15°C cold water, and then dried under vacuum. The average particle size of microbeads in 15°C cold water was 210 microns. When the temperature was raised to 37°C, the beads shrank and became opaque. After washing 1 g of microbeads with sterile water thoroughly, the beads were dispersed in 5 ml of DMEM containing 10% FCS which was warmed to 37°C, and mouse dermal fibroblasts were added to bring the cell concentration to about $1 \times 10^7$/ml. Then they were transferred into a glass beaker, and incubated at 37°C for 4 hours in a $CO_2$ gas incubator agitated by a magnetic stirrer. Later, microbeads were removed from the glass beaker, and the surface of the microbeads was washed twice with the PBS which was warmed to 37°C, and then the microbeads were immobilized with 1% glutaraldehyde-PBS which was warmed to 37°C. After washing the immobilized microbeads with distilled water, they were dehydrated with alcohol, dried by the critical point drying technique, and gold palladium was deposited by vapro process. Cell adhesion was observed by scanning electron microscopy. Alternatively, microbeads taken out after 4 hours incubation were allowed to stand for 20 minutes in the 15°C medium, and the medium was discarded. The surface of the microbeads was washed twice with 15°C PBS, and the microbeads were immobilized with the 1% glutaraldehyde-PBS which was cooled to 15°C. The immobilized microbeads were examined by scanning electron microscopy by the same procedure as before, to observe cell adhesion. As a result, it was found that the number of cells on the surface of microbeads immobilized at 15°C was extremely small compared to the number of cells on the surface of microbeads immobilized at 37°C. Almost no cells were found attached to the surface of microbeads immobilized at 15°C. This evidence suggests that cells were detached entirely from the surface of the beads by cooling the microbeads.

## Example 19

Using the dish coated with the mixture of collagen and PNIPAAm (collagen/PNIPAAm = 1.0/1.0) at a thickness of about 0.9 um which is prepared in Example 7, the human dermal fibroblasts were cultured in the similar manner to Example 7. After a 5-day culture, the cells fully covered the dish. When the dish was withdrawn from a 37°C incubator and left at an ambient temperature, the cell sheet spontaneously detached from the dish and the suspended cell sheet was washed twice with fresh medium in order to remove dissolved PNIPAAm and collagen from the cell sheet. Thereafter, the cell sheet was transferred to a new non-anchorage hydrophobic 35 mm dish (Falcon Co.) containing 2 ml of fresh culture medium, and was cultured under suspension for 2 days until a spheroidal cell cluster was obtained. The size of the obtained spheroidal cell cluster was about 1 mm and the cell density was in the order of $10^9$/ml.

On the other hand, the obtained spheroidal fibroblast cluster was cultured on the non-anchorage dish in a $CO_2$ incubator (air/5% $CO_2$) for an extra 20 days and then the cell cluster was transferred to the plastic hydrophilic dish (Falcon Co.) and was cultured in similar conditions to the above-mentioned. After a 2-day culture, the cell cluster reattached to the hydrophilic dish surface and reproliferated after a 10-day culture. This evidence demonstrates that the cell cluster has proliferation activity even after a long preservation.

For controls, using the dish coated only with collagen prepared in Example 8, in a similar manner to the above-mentioned conditions, the human dermal fibroblasts were cultured. In order to detach the cell sheet which fully covered the dish after a 4-day culture, the prior cell detachment procedures were carried out. The old culture medium was discarded from the dish and 2 ml of PBS was poured into the dish to wash the surface of the cells for removal of trypsin inhibitors contained in the medium and then the PBS was discarded. Then 2 ml of trypsin/EDTA solution (0.05% trypsin, 0.53 mM EDTA) was added to wash the surface of the cells, and the trypsin/EDTA solution was discarded. Again 2 ml of fresh trypsin/EDTA solution was added and the solution, except 0.5 ml, was discarded. Then the dish was incubated at $37^{\circ}$ C for 10 minutes. Using a microscope, complete detachment of the cells from the dish was confirmed, but the detached cells were isolated from each other and neither made a cell sheet nor a cell cluster. Accordingly, by this cell detachment method, it was impossible to prepare these cell sheets and cell clusters.

## Example 20

The fibroblast sheet which was obtained by the detachment procedure in Example 16, was transferred to a non-anchorage hydrophobic dish and cultured in a similar manner to Example 19. After a 2-day culture the cell sheet completely changed to a cluster whose size was about 1 mm. After the cell cluster was preserved on the hydrophobic dish at $37^{\circ}$ C in a $CO_2$ incubator (air/5% $CO_2$ for 3 months, the cluster was again transferred to a new hydrophilic dish and was cultured. After 2 days, the cluster attached to the dish and started to reproliferate. This evidence shows that the cell cluster continues to live even after 3 months of preservation.

## Claims

1. A cell culture substrate comprising a temperature-responsive polymeric compound having a lower LCST than the culture temperature.

2. The cell culture substrate of Claim 1, wherein the said polymeric compound is selected from the group comprising poly-N-substituted acrylamide derivatives, poly-N-substituted methacrylamide derivatives, their copolymers, polyvinylmethyl ether and partially acetylated polyvinylalcohol.

3. The cell culture substrate of Claim 1, further comprising substances which effectively control cell functions.

4. The cell culture substrate of Claim 3 wherein said substance which effectively controls cell function is selected from the group comprising extracellular matrix, gelatin, lectin, anchorage oligopeptide and adhesive protein isolated from shellfish.

5. The cell culture substrate of Claim 4 wherein the said extracellular matrix is selected from the group consisting of collagen, fibronectin, vitronectin, laminin, proteoglycan, glycosaminoglycan and thrombospondin.

6. The cell culture substrate of Claim 1 wherein the said polymeric compound is coated on the surface of the supporting material.

7. The cell culture substrate of Claim 1 wherein the said polymeric compound is graft-copolymerized on the surface of the supporting material.

8. The cell culture substrate of Claim 1 wherein the said polymeric compound has a crosslinked structure.

9. The cell culture substrate of Claim 1 wherein the shape at the cell culture temperature is a film, sheet, particle, fiber, flake, sponge or microbead.

10. The cell culture substrate of Claim 3 wherein said polymeric compound and said substance which effectively controls cell function are coated on the surface of the supporting material as a homogeneous mixture.

11. The cell culture substrate of Claim 3, wherein said polymeric compound and said substance which effectively controls cell function are coated on the surface of the supporting material in a sequential manner.

12. A method for preparation of the cell culture substrate of Claim 3 comprising coating said substance which effectively controls cell function and said temperature-sensitive polymeric compound having lower LCST than the culturing temperature on the surface of the support member.

13. A method for fabricating cell sheets comprising using a cell culture substrate containing at least partially a temperature-responsive polymer having lower LCST than the cell culture temperature, lowering the temperature to lower than LCST after proliferation of cells, and removing the proliferated cells from the supporting material.

14. The method of Claim 13, wherein said temperature-responsive polymer is selected from poly-N-substituted acrylamide derivatives, its copolymers, or partially acetylated polyvinyl alcohols or polymethylvinyl ethers.

15. The method of Claim 13, wherein said cell culture substrate further comprises a substance which effectively controls cell function.

16. Cell sheets prepared by the method of Claim 13.

17. A method for preparation of cell clusters, comprising culturing cells on a substrate containing temperature-sensitive polymeric compound having a lower LCST than at least the cell culturing temperature, and lowering the temperature below the LCST after propagation of cells to release the propagated cells from the substrate.

18. The method of Claim 17, wherein said substrate further comprises a substance which effectively controls cell function.

19. The method of Claim 27, wherein said temperature-sensitive polymeric compound is selected from poly-N-substituted methacrylamide derivatives, their copolymers, polymethylvinyl ethers or partially acetylated polyvinyl alcohols.

20. Cell clusters prepared by the method of Claim 17.

European Patent Office

EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| Y | PATENT ABSTRACTS OF JAPAN, vol. 8, no. 205 (C-243)[1642], 19th September 1984; & JP-A-59 95 930 (KOGYO GIJUTSUIN (JAPAN)) 02-06-1984 * The entire abstract * | 1-20 | C 12 N 5/00 |
| Y | WO-A-8 808 448 (M. BAY) * The claims; page 2, line 30 - page 3, line 15; page 5, lines 1-17; page 7, lines 30-33; page 9, lines 5-11; page 10, lines 10-29; page 11, lines 3-5; page 12, lines 18-26 * | 1-20 | |

TECHNICAL FIELDS
SEARCHED (Int. Cl.5)

C 12 N

The present search report has been drawn up for all claims

| Place of search | Date of completion of the search | Examiner |
|---|---|---|
| THE HAGUE | 26-06-1990 | RYCKEBOSCH A.O.A. |